(19) Europäisches Patentamt
European Patent Office
Office européen des brevets

(11) **EP 2 186 478 A1**

(12) **EUROPÄISCHE PATENTANMELDUNG**

(43) Veröffentlichungstag:
**19.05.2010 Patentblatt 2010/20**

(51) Int Cl.:
***A61B 5/103*** *(2006.01)*

(21) Anmeldenummer: **08019876.5**

(22) Anmeldetag: **14.11.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **HASOMED Hard- und Software für Medizin GmbH**
**39114 Magdeburg (DE)**

(72) Erfinder:
• **Kauert, Ralf, Dr.rer.nat.**
**39128 Magdeburg (DE)**

• **Halfpaap, Josef, Dipl.-Ing.**
**39114 Magdeburg (DE)**
• **Weber, Matthias, Dipl.-Ing.**
**39175 Biederitz (DE)**
• **Liedecke, Wolfgang, Dr.-Ing.**
**06502 Thale-Warnstedt (DE)**

(74) Vertreter: **Fischer, Volker**
**Fritz-Reuter-Strasse 7**
**39108 Magdeburg (DE)**

(54) **Verfahren und Vorrichtung zur Analyse des menschlichen Gangzyklus**

(57) Vorrichtung und Verfahren zur Analyse des menschlichen Gangzyklus, **dadurch gekennzeichnet, dass** ein Näherungssensor (5) zur Ermittlung der Fuß-Fuß-Passage an den Füßen angebracht ist. Die Auswertung der ermittelten Messwerte erfolgt in der Ganganalyseeinrichtung (4), wobei mit der Fuß-Fuß-Passage ein initialer Zeitpunkt zur Zuordnung der Gangphasen gewonnen wird.

Figur 2

EP 2 186 478 A1

**Beschreibung**

Die Erfindung betrifft ein Verfahren und eine Vorrichtung zur Analyse des menschlichen Gangzyklus.

Verfahren und Vorrichtungen der vorstehend beschriebenen Gattung dienen zur Analyse des menschlichen Gangzyklus. Die Messung und Beurteilung des menschlichen Ganges erfolgt u. a. in der Orthopädie, in der neurologischen Rehabilitation, in der Sportmedizin und beim Anpassen von Hilfsmitteln wie Orthesen und Prothesen. Anhand des Gangbildes kann einerseits der Verlauf des Rehabilitationsprozesses beurteilt werden, andererseits dient es der Steuerung und Begründung therapeutischer Maßnahmen. Eine weitere mögliche Anwendung ist die Früherkennung pathologischer Veränderungen im Gangbild.

Mit dem wohl am weitesten verbreiteten Verfahren zur Ganganalyse, der rein visuellen Beurteilung des Ganges bei einer Laufprobe, sind Fehlbewegungen und -belastungen oftmals nicht zu erkennen. Andererseits ist diese Methode sehr subjektiv, da das Ergebnis der Analyse von der jeweiligen Qualifikation des Therapeuten oder Orthopädietechnikers abhängt. Insbesondere ist aber mit dieser Methode eine frühzeitige Erkennung bis dahin nicht sichtbarer Veränderungen nicht möglich.

In jüngster Zeit hat die quantitative Bestimmung der Dynamik des menschlichen Stütz- und Bewegungsapparates mit technischen Mitteln an Bedeutung gewonnen. Bekannt sind kinematische Ganganalysesysteme, bei denen die Bewegungen von befestigten Markern mit Hilfe von Kameras oder Ultraschall festgehalten werden. Die daraus bestimmten Kurven geben Auskunft über das Gehverhalten des zu untersuchenden Patienten. Eine weitere Gruppe an Ganganalyseverfahren beruht auf der Messung des Druckes unter den Füßen.

In letzter Zeit sind auch Inertialsensormesssysteme im Gespräch, die die Bewegung des Patienten mit Hilfe der gemessenen Beschleunigungen und Winkelgeschwindigkeiten rekonstruieren sollen.

Der Gang ist ein sehr komplexes Geschehen, das sich nur schwer komplett beschreiben und analysieren lässt. Einen möglichen Zugang liefert die Modellvorstellung, dass der Gang ein zyklischer Prozess ist. Er besteht aus einer Abfolge von immer wiederkehrenden Zuständen, den Gangphasen, und Zustandsübergängen, den Gangereignissen. Die Untersuchung der Gangphasen und Gangereignisse liefert Anhaltspunkte für pathologische Veränderungen. Eine turnusmäßige Messung kann Rückschlüsse auf den Erfolg von therapeutischen Behandlungen liefern. Um geringe Veränderungen des Gangbildes aufspüren zu können, wäre jedoch eine präzise, durch Software gestützte, automatische Erkennung der Gangphasen und Gangereignisse von Vorteil.

Die einfachste Einteilung des Gangzyklus in Gangphasen ist die Unterteilung in Standphase und Schwungphase. Während der Standphase steht der Fuß auf dem Boden, während der Schwungphase bewegt sich der Fuß nach vorn. Sowohl Standphase als auch Schwungphase können noch weiter unterteilt werden. Dazu wurden in der Fachliteratur verschiedene Gangphaseneinteilungssysteme vorgestellt. Ein Beispiel dafür ist das Gangphaseneinteilungssystem von Perry (Jaquelin Perry: "Ganganalyse - Norm und Pathologie des Gehens", Urban und Fischer). Dieses System unterteilt den Gangzyklus in 8 Phasen.

Die Ganganalyse ist bereits bekannt. So bietet die DE 69431296 T2, in einer Patentfamilie unter anderem mit der EP 0703752 B1 und der US 6010465 A, eine Vorrichtung zur Überprüfung und zum Training der Balance des menschlichen Bewegungsapparates in unterschiedlichen Positionen wie Stehen oder Sitzen und Bewegungen wie Aufsteigen oder Aufstehen. Dabei werden die jeweils auftretenden Druckkräfte gemessen und verglichen. Trainiert werden sollen Koordinationsfähigkeit und Gleichgewicht, wozu eine Auswertung der gemessenen Kraftwirkungen erfolgt.

Die Möglichkeit, komplexe Bewegungsabläufe abzubilden, besteht nicht.

In der US 6010465 A ist eine Vorrichtung und ein Verfahren zur Ganganalyse beschrieben. Die Vorrichtung bedient sich eines Laufbandes, wobei unter der Lauffläche Sensoren angebracht sind, im einfachsten Fall zwei Druckplatten, die die Belastung feststellen. Dadurch ist es in einer bevorzugten Ausgestaltung möglich, die Druckwirkung der Fußfläche durch eine große Anzahl von Sensoren auch in höherer Auflösung zu ermitteln. Somit kann das Aufsetzen der Ferse oder das Abstoßen des Fußballens ebenso registriert werden wie Gewichtsverlagerungen des Standfußes.

Über den Bewegungsablauf des Schwungsbeins ist jedoch nichts bekannt, Rückschlüsse auf dessen Position, mit Bezug zur am Standfuß festgestellten Gewichtsverlagerungen, sind nicht möglich.

In der US 6645126 B1 wird ein Gehtrainer offenbart. Dieser basiert auf einem Laufband, das derart ausgeführt ist, dass die Schrittlänge über die Bandlaufgeschwindigkeit vorgegeben werden kann. In einer vorteilhaften Weiterentwicklung ist die Vorrichtung mit Sensoren ausgerüstet, die das Auftreffen des linken oder des rechten Fußes registrieren.

Nachteilig ist, dass einzig das Auftreffen der Füße auf dem Laufband registriert wird, während der gesamte Bewegungsablauf der Füße und Beine ohne Kontrolle bleibt.

Die WO 87/01574 A1 beschreibt eine Vorrichtung zur Überprüfung der Phasen des Gangs. Es handelt sich dabei um eine computergesteuerte Plattform, die auf den Druck einer gehenden Fußsohle reagiert.

Die Erfindung besteht in einem besonderen Aufbau der drucksensitiven Matte. Die Ermittlung weiterer Daten als der Druckwirkung des auftretenden Fußes ist mittels der offenbarten Lösung nicht möglich.

Das Gebrauchsmuster DE 9017 709 U1 beschreibt eine Vorrichtung zur automatischen Ganganalyse, die auf einem Laufband basiert. Hierbei weist das Laufband Lamellen auf, die miteinander verbunden und mit Sensoren ausgestattet sind. Diese Sensoren gestatten die Ermittlung von Vertikal- und Horizontalkräften.

Auch bei dieser Lösung zeigt sich als Nachteil, dass ohne auf den Sensoren aufliegenden Fuß keinerlei Informationen über dessen Position verfügbar sind.

Ein weiterer Nachteil der Ganganalysesysteme nach dem Stand der Technik ist, dass sie entweder über keine oder nur eine grobe und unzuverlässige automatische Gangphasenerkennung verfügen. Bei Markersystemen besteht die technische Schwierigkeit, dass diese meist nur eine Körperseite betrachten und in der Regel auch mit nur 50 Hz Messrate arbeiten, was eine automatische Erkennung der Gangphasen erschwert. Bei den Systemen, die die Druckverteilung unter den Fußsohlen messen, können keine Aussagen getroffen werden über die Vorgänge innerhalb der Schwungphase.

Ein Problem bei der automatischen Bestimmung der Gangphasen ist die Erkennungssicherheit und Robustheit gegenüber Störungen.

Beim Einsatz von Inertialsensoren zur Ganganalyse besteht das zusätzliche Problem, dass Inertialsensoren keine Winkel und Ortskoordinaten messen können, sondern nur Winkelgeschwindigkeiten und Beschleunigungen. Um von den Messwerten auf Winkel und Ortskoordinaten zu kommen, ist ein aufwändiger Berechnungsalgorithmus notwendig.

$$v = \int a \cdot dt + C_v, \quad s = \int v \cdot dt + C_s$$

Dieser basiert auf mehrfacher numerischer Integration, der jedoch die nachteilige Eigenschaft anhaftet, dass sich Fehler, die durch Messungenauigkeiten oder Verfahrensfehler entstehen, akkumulieren können. Man spricht dabei von Integrationsdrift. Im Extremfall können die Ergebnisse komplett unbrauchbar sein, ohne dass dieses bemerkt wird. Deshalb ist es notwendig, in bestimmten Intervallen Korrekturen durchzuführen.

Der Erfindung liegt deshalb die Aufgabe zugrunde, ein Verfahren und eine Vorrichtung gemäß dem Oberbegriff der Erfindung zu einem robusten, erkennungssicheren Verfahren und einer zu dessen Durchführung erforderlichen Vorrichtung weiterzubilden, eine Gangphasenerkennung zu realisieren.

Die Lösung der Aufgabe erfolgt durch eine Vorrichtung zur Analyse des menschlichen Gangzyklus, wobei ein Näherungssensor zur Ermittlung der Fuß-Fuß-Passage an den Füßen angebracht ist. Der Näherungssensor ist als ein Magnetfeldsensor an dem einen Fuß und Permanentmagnet an dem anderen Fuß ausgeführt. In einer Ausgestaltung der Lösung ist der Näherungssensors an den Schuhen befestigt und die Messwerte werden auf die Auswerteeinrichtung übertragen.

Teil der Lösung ist zudem eine Differenzierungsvorrichtung zur Eliminierung von Magnetfeldstörungen. Die Messwerte werden dazu vom Näherungssensor auf die Differenzierungsvorrichtung übertragen.

Das Verfahren zur Analyse des menschlichen Gangzyklus, ist **dadurch gekennzeichnet, dass** eine zusätzliche Information über einen initialen Zeitpunkt im Ablauf des Gangzyklus gewonnen wird, durch den die Zuordnung der mittels bekannter Methoden gewonnenen Messwerte zu den einzelnen Phasen innerhalb des Gangzyklus erfolgt und dass als initialer Zeitpunkt die Fuß-Fuß-Passage ermittelt wird.

Die Aufgabe wird weiterhin gelöst durch ein Verfahren, bei dem der Zeitpunkt der Fuß-Fuß-Passage als Signal des an den Füßen befestigten Näherungssensors während des Gehens als Messsignal erfasst wird. Das Signal des als Magnetfeldsensors ausgeführten Nährungssensors wird aus einem beim Gehen veränderlichen Magnetfeld gewonnen.

Mit der Differenzierung des Messsignals des Näherungssensors werden Magnetfeldstörungen eliminiert.

Bei der Rekonstruktion der Sensortrajektorien, d.h. der Ermittlung von Winkeln und Ortskoordinaten als Funktion der Zeit aus den mittels Inertialsensoren gemessenen Winkelgeschwindigkeiten und Beschleunigungen, können, wie bereits erläutert, Fehler durch mehrfache numerische Integration entstehen, die einen erheblichen Einfluss auf das Ergebnisse haben. Insbesondere kann der Effekt auftreten, dass die Ortskoordinaten des linken und des rechte Fußes durch die Integrationsdrift scheinbar auseinanderdriften. Die erfindungsgemäße Bestimmung des Zeitpunktes der Fuß-Fuß-Passage erlaubt, das Rekonstruktionsverfahren durch eine zusätzliche Zwangsbedingung zu stabilisieren. Es kann die Annahme gemacht werden, dass zum Zeitpunkt der Fuß-Fuß-Passage $t_{FFP}$ die Koordinate in Wegrichtung, die in der Regel mit x bezeichnet wird, für beide Füße den gleichen Wert haben muss:

$$x_L(t_{FFP}) = x_R(t_{FFP}).$$

Damit kann die Integrationsdrift bei der Umrechnung der von Inertialsensoren als Messwerte gelieferten Winkelgeschwindigkeiten und Beschleunigungen zu Winkeln und Ortskoordinaten wirksam korrigiert werden.

Ein Vorteil der Erfindung liegt darin, dass sich durch das beschriebene Verfahren und die beschriebene Vorrichtung der Zeitpunkt der Fuß-Fuß-Passage automatisch bestimmen und mit verschiedenen etablierten Ganganalyseverfahren kombinieren lässt. Beispielsweise kann bei einem Druckmesssohlensystem eine Aussage über den Verlauf der Schwungphase gewonnen werden. Bei einem Markersystem kann der Zeitpunkt der Fuß-Fuß-Passage bestimmt werden, während sich die Beine gerade überdecken und die Markerverfolgung zu fehlerhaften Ergebnissen kommt.

Weitere Einzelheiten, Merkmale und Vorteile der Erfindung ergeben sich aus der nachfolgenden Beschreibung eines Ausführungsbeispiels mit Bezugnahme auf die zugehörigen Zeichnungen. Es zeigen:

Figur 1  ein Verfahrensschema bei der Bestimmung der Fuß-Fuß-Passage,

Figur 2  ein Ausführungsbeispiel zur Bestimmung der Fuß-Fuß-Passage mit an Schuhen angebrachten Permanentmagneten und Magnetfeld-sensor,

Figur 3  ein Schema zur Festlegung des Fußwinkels,

Figur 4  einen zeitlichen Verlauf des Magnetfeldsensorsignales und des Winkels zwischen Fußsohle und Erdboden in Grad (° Fußwinkel),

Figur 5  einen zeitlichen Verlauf des differenzierten Magnetfeldsensorsignales und des Fußwinkels in Rad und

Figur 6  einen zeitlichen Verlauf des differenzierten Magnetfeldsensorsignales und des Fußwinkels in Rad sowie markierte Fuß-Fuß-Passagen.

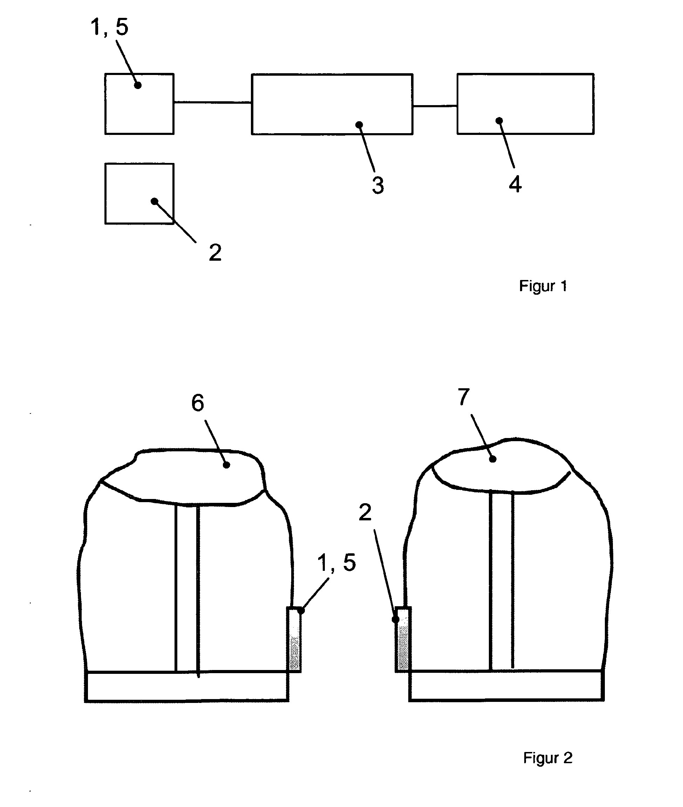

Figur 1 zeigt das Verfahrensschema bei der Bestimmung der Fuß-Fuß-Passage. Der Magnetfeldsensor 1 detektiert eine Magnetfeldänderung, die durch die Annäherung des Permanentmagneten 2 hervorgerufen wurde. Da bedingt durch das Erdmagnetfeld oder andere Magnetfelder störende Einflüsse auftreten, werden diese Störungen in der Differenzierungsvorrichtung 3 herausgefiltert und das nun von Störsignalen freie Signal für eine Fuß-Fuß-Passage an eine Ganganalyseeinrichtung 4 gemäß dem Stand der Technik übergeben.

In der Figur 2 ist die bevorzugte Ausgestaltung der Erfindung dargestellt. Hierbei ist der Näherungssensor 5 als Permanentmagnet 2 und Magnetfeldsensor 1 ausgeführt und beide Teile des Näherungssensor 5 an den beiden Schuhen 6, 7 in Höhe des Knöchels befestigt. Dabei trägt der rechte Schuh 7 den Permanentmagneten 2 und der linke Schuh 6 den Magnetfeldsensor 1. Der Permanentmagnet 2 beeinflusst den Magnetfeldsensor 1 , sobald beide in geringer Entfernung aneinander vorbeigeführt werden. Das geschieht sowohl, wenn der linke Schuh 6 mit dem Magnetfeldsensor 1 den Fuß des Standbeins bekleidet, als auch im umgekehrten Fall, wenn der Fuß des Standbeins im rechten Schuh 7 mit dem Permanentmagneten 2 steckt. Die in der Figur 2 nicht dargestellte Differenzierungsvorrichtung 3 filtert aus dem Signal die Störsignale heraus und stellt ein sicheres Signal der Fuß-Fuß-Passage bereit.

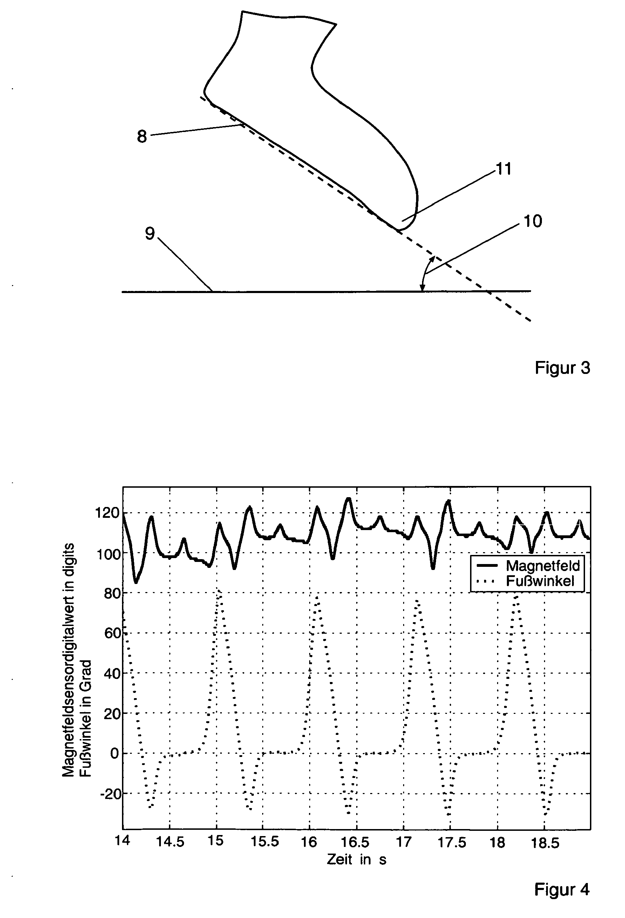

In Figur 3 ist die Definition des Fußwinkels 10 dargestellt, der zwischen Boden 9 und Fußsohle 8 gemessen wird. Ein Fußwinkel 10 mit positivem Vorzeichen gibt eine Fußstellung mit zum Boden 9 gerichteter Fußspitze 11 an.

Figur 4 zeigt ein Diagramm mit Magnetfeldsensorsignal und Winkel zwischen Fußsohle 8 und Boden 9 in Grad (Fußwinkel) eines Fußes in einem punktierten Graphen sowie einen Wert für die Stärke des Magnetfelds, dargestellt als durchgezogene Kurve. Der Magnetfeldsensorsdigitalwert ist in Digits, den Digitalisierungsstufen des Analog-Digital-(AD-)Wandlers, angegeben. Ein Digit ist dabei die kleinste Digitalisierungsstufe des AD-Wandlers. Diese Einheit entspricht den praktischen Anforderungen, da die Angabe der magnetischen Flussdichte in Tesla irrelevant wäre.

Im Diagramm ist zwar visuell erkenn- und zuordenbar, dass im Moment des vollständig aufgesetzten Fußes des Standbeins das andere Bein vorbeischwingt, allerdings ist das Signal von vielen Störsignalen überlagert die eine sichere automatische Auswertung erschweren.

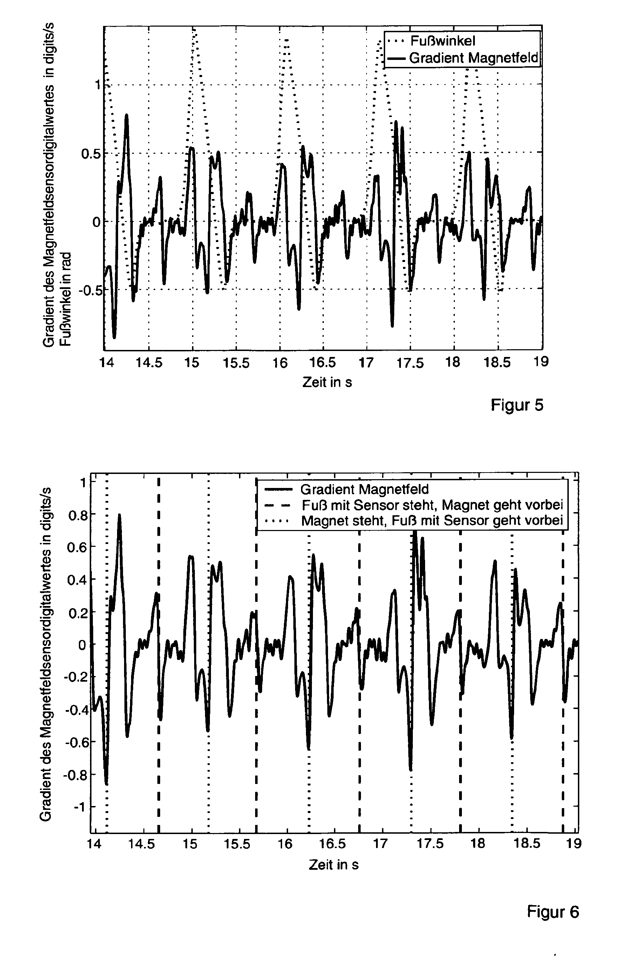

Figur 5 zeigt dieselbe Situation, wie sie in Figur 4 dargestellt ist, jedoch nachdem das Signal tiefpassgefiltert wurde und unter Einsatz der Differenzierungsvorrichtung 3 differenziert wurde. Diese eliminiert die Störsignale weitgehend, so dass ein klares Signal zur Kennzeichnung der Fuß-Fuß-Passage entsteht, das unter Einbeziehung einer Zeitmessung den Zeitpunkt der Fuß-Fuß-Passage angibt. Diese Information kann in einer Ganganalyse gemäß dem Stand der Technik vorteilhaft weiterverarbeitet werden. Gezeigt werden in der Figur 5 ein differenziertes Magnetfeldsensorsignal als Gradient des Magnetfeldsensordigitalwertes in Digits pro Sekunde und der Fußwinkel in Rad.

In Figur 6 sind zusätzlich zur Darstellung aus Figur 5 die durch einen in der Ganganalyseeinrichtung 4 ablaufenden Algorithmus festgestellten Zeitpunkte der Fuß-Fuß-Passagen durch senkrechte Linien markiert. Gestrichelte Linien markieren eine Fuß-Fuß-Passage, bei der der Fuß mit dem Magnetfeldsensor 1 steht und der Fuß mit dem Permanentmagneten 2 vorbeigeführt wird. Demgegenüber stehen gepunktete Linien für den umgekehrten Fall, wenn der Fuß mit dem Permanentmagneten 2 steht und der Fuß mit dem Magnetfeldsensor 1 vorbeigeführt wird.

Liste der verwendeten Bezugszeichen

1 -   Magnetfeldsensor
2 -   Permanentmagnet
3 -   Differenzierungsvorrichtung
4 -   Ganganalyseeinrichtung
5 -   Näherungssensor
6 -   linker Schuh
7 -   rechter Schuh
8 -   Fußsohle
9 -   Boden
10 -  Fußwinkel
11 -  Fußspitze

**Patentansprüche**

**1.** Vorrichtung zur Analyse des menschlichen Gangzyklus,
**dadurch gekennzeichnet, dass**
ein Näherungssensor (5) zur Ermittlung der Fuß-Fuß-Passage an den Füßen angebracht ist.

**2.** Vorrichtung nach Anspruch 1,
**dadurch gekennzeichnet, dass**
der Näherungssensor (5) als ein Magnetfeldsensor (1) an dem einen Fuß und Permanentmagnet (2) an dem anderen Fuß ausgeführt ist.

**3.** Vorrichtung nach Anspruch 1 oder 2,
**dadurch gekennzeichnet, dass**
der Näherungssensors (5) an den Schuhen (6, 7) befestigt ist und die Messwertübertragung auf die Ganganalyseeinrichtung (4) erfolgt.

**4.** Vorrichtung nach einem der Ansprüche 1 bis 3,
**dadurch gekennzeichnet, dass**
eine Differenzierungsvorrichtung (3) zur Eliminierung von Magnetfeldstörungen vorgesehen ist.

**5.** Vorrichtung nach einem der Ansprüche 1 bis 4,
**dadurch gekennzeichnet, dass**
eine Messwertübertragung vom Näherungssensor (5) auf die Differenzierungsvorrichtung (3) erfolgt.

**6.** Verfahren zur Analyse des menschlichen Gangzyklus,
**dadurch gekennzeichnet, dass**
eine zusätzliche Information über einen initialen Zeitpunkt im Ablauf des Gangzyklus gewonnen wird, durch den die Zuordnung der mittels bekannter Methoden gewonnenen Messwerte zu den einzelnen Phasen innerhalb des Gangzyklus erfolgt und dass als initialer Zeitpunkt die Fuß-Fuß-Passage ermittelt wird.

**7.** Verfahren nach Anspruch 6,
**dadurch gekennzeichnet, dass**
der Zeitpunkt der Fuß-Fuß-Passage als Signal des an den Füßen befestigten Näherungssensors (5) während des Gehens als Messsignal erfasst wird.

**8.** Verfahren nach Anspruch 6 oder 7,
**dadurch gekennzeichnet, dass**
das Signal des als Magnetfeldsensors (1) ausgeführten Nährungssensors (5) aus einem beim Gehen veränderlichen Magnetfeld gewonnen wird.

**9.** Verfahren nach einem der Ansprüche 6 bis 8,
**dadurch gekennzeichnet, dass**
mit der Differenzierung des Messsignals des Näherungssensors (5) Magnetfeldstörungen eliminiert werden.

**10.** Verfahren nach einem der Ansprüche 6 bis 9,
**dadurch gekennzeichnet, dass**
der Zeitpunkt der Fuß-Fuß-Passage zur Korrektur der Integrationsdrift bei der Umrechnung der von Inertialsensoren als Messwerte gelieferten Winkelgeschwindigkeiten und Beschleunigungen zu Winkeln und Ortskoordinaten verwendet wird.

Figur 1

Figur 2

Figur 3

Figur 4

Figur 5

Figur 6

Europäisches
Patentamt

European
Patent Office

Office européen
des brevets

# EUROPÄISCHER RECHERCHENBERICHT

**Nummer der Anmeldung**

EP 08 01 9876

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2007/123806 A1 (BOUVIER ALAIN [FR] ET AL) 31. Mai 2007 (2007-05-31) | 1-5 | INV. A61B5/103 |
| A | * Zusammenfassung; Abbildungen * <br> * Seite 2 - Seite 4 * <br> ----- | 6-10 | |
| X | US 4 371 945 A (KARR LAWRENCE J [US] ET AL) 1. Februar 1983 (1983-02-01) | 1 | |
| A | * Zusammenfassung; Abbildung 2 * <br> * Spalte 4, Zeile 33 - Zeile 65 * <br> * Spalte 6, Zeile 40 - Zeile 56 * <br> * Spalte 7, Zeile 14 - Zeile 42 * <br> ----- | 6-10 | |
| X | US 6 122 960 A (HUTCHINGS LAWRENCE J [US] ET AL) 26. September 2000 (2000-09-26) <br> * Zusammenfassung; Abbildungen * | 1 | |
| A | * Spalte 4 - Spalte 5 * <br> * Spalte 26, Zeile 37 - Zeile 43 * <br> * Spalte 8, Zeile 20 - Spalte 9, Zeile 16 * <br><br> ----- | 6-10 | |
| A | CA 1 193 436 A1 (ZIEMLINSKI ISABELLA; SITARSKI HENRYK) 17. September 1985 (1985-09-17) * Seite 2 - Seite 5; Abbildungen 1-3 * ----- | 1,2,6-10 | **RECHERCHIERTE SACHGEBIETE (IPC)** <br><br> A61B |
| A | US 2002/040601 A1 (FYFE KENNETH R [CA] ET AL) 11. April 2002 (2002-04-11) * Absatz [0034] - Absatz [0035]; Abbildungen * ----- | 1,2,6-10 | |
| A | WO 2007/058526 A (XSENS TECHNOLOGIES B V [NL]; UNIV TWENTE [NL]; SLYCKE PER JOHAN [NL];) 24. Mai 2007 (2007-05-24) * Seite 4 - Seite 6; Abbildungen * ----- | 6-10 | |

Der vorliegende Recherchenbericht wurde für alle Patentansprüche erstellt

| Recherchenort | Abschlußdatum der Recherche | Prüfer |
|---|---|---|
| München | 28. April 2009 | Mundakapadam, S |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

   ....................................................................................................
& : Mitglied der gleichen Patentfamilie, übereinstimmendes
   Dokument

EPO FORM 1503 03.82 (P04C03)

| | Europäisches<br>Patentamt<br>European<br>Patent Office<br>Office européen<br>des brevets |
|---|---|

Nummer der Anmeldung

EP 08 01 9876

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

```
Siehe Ergänzungsblatt B
```

☒ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

EP 08 01 9876

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

```
    1. Ansprüche: 1-5

        Vorrichtung zur Analyse des menschlichen Gangzyklus,
        umfassend einen Näherungssensor in Form eines
        Magnetfeldsensors
                        ---


    2. Ansprüche: 6-10

        Verfahren zur Analyse des menschlichen Gangzyklus, bei der
        die Fuß-Fuß-Passage als initialer Zeitpunkt ermittelt wird
        und als zusätzliche Information zur Zuordnung von Messwerten
        verwendet wird
                        ---
```

**ANHANG ZUM EUROPÄISCHEN RECHERCHENBERICHT
ÜBER DIE EUROPÄISCHE PATENTANMELDUNG NR.**

EP 08 01 9876

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-04-2009

| Im Recherchenbericht angeführtes Patentdokument | Datum der Veröffentlichung | Mitglied(er) der Patentfamilie | Datum der Veröffentlichung |
|---|---|---|---|
| US 2007123806 A1 | 31-05-2007 | EP 1673590 A2<br>FR 2860700 A1<br>WO 2005034751 A2 | 28-06-2006<br>15-04-2005<br>21-04-2005 |
| US 4371945 A | 01-02-1983 | KEINE | |
| US 6122960 A | 26-09-2000 | KEINE | |
| CA 1193436 A1 | 17-09-1985 | KEINE | |
| US 2002040601 A1 | 11-04-2002 | US 6301964 B1 | 16-10-2001 |
| WO 2007058526 A | 24-05-2007 | EP 1959831 A1<br>NL 1030440 C2 | 27-08-2008<br>21-05-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69431296 T2
- EP 0703752 B1
- US 6010465 A
- US 6645126 B1
- WO 8701574 A1
- DE 9017709 U1